# EUROPEAN PATENT SPECIFICATION

(11) **EP 4 371 609 B1**
(45) Date of publication and mention of the grant of the patent: **16.09.2026**
(21) Application number: 22841413.2
(22) Date of filing: 13.07.2022
(51) Int. Cl.: A61N 5/10, H05H 3/06

(54) **NEUTRON CAPTURE THERAPY SYSTEM**
NEUTRONENEINFANGTHERAPIESYSTEM
SYSTÈME DE THÉRAPIE CAPTURE DE NEUTRONS

(30) Priority: 16.07.2021 CN 202110807235
(43) Date of publication of application: 22.05.2024
(73) Proprietor: Neuboron Therapy System Ltd., Xiamen, Fujian 361026 (CN)
(72) Inventor: SHU, Di-yun, Nanjing, Jiangsu 211112 (CN); GONG, Qiu-ping, Nanjing, Jiangsu 211112 (CN)
(74) Representative: Gunzelmann, Rainer
(86) International application number: PCT/CN2022/105433
(87) International publication number: WO 2023/284780

(56) References cited:
- EP-A1- 3 666 337
- EP-A1- 3 685 883
- EP-B1- 2 754 336
- WO-A1-2010/019584
- CN-A- 102 695 544
- CN-A- 104 039 391
- CN-A- 104 780 975
- CN-A- 105 120 952
- CN-A- 109 464 752
- CN-U- 213 159 020
- US-A1- 2009 289 194

## Description

### TECHNICAL FIELD

The present disclosure relates to a radiation irradiation system, in particular to a neutron capture therapy system.

### BACKGROUND

With the development of atomic science, radiation therapy, such as cobalt-60 radiation therapy, linear accelerator therapy and electron beam radiation therapy, has become one of the main means of cancer therapy. However, the traditional photon or electron therapy is limited by physical conditions of the radiation itself, which also damages a large amount of normal tissues in the beam path while killing tumor cells. In addition, due to different sensitivity of tumor cells to radiation, the traditional radiation therapy is often ineffective for therapy of malignant tumors with relatively high radiation resistance (e. g., glioblastoma multiforma and melanoma).

In order to reduce radiation damage to normal tissues surrounding tumors, the concept of target therapy in chemotherapy is applied to radiation therapy; for tumor cells with high radiation resistance, radiation sources with high relative biological effectiveness (RBE), such as proton therapy, heavy particle therapy and neutron capture therapy, are also actively developed at present. The neutron capture therapy combines the above two concepts, such as boron neutron capture therapy, which provides a better selection of cancer therapy than conventional radiation therapy by means of specific aggregation of a boron-containing drug in tumor cells, and in conjunction with precise neutron beam regulation.

Various radiations are generated during the radiation therapy, for example, neutrons and photons with energies ranging from low to high are generated during the boron neutron capture therapy, and these radiations may cause different degrees of damage to normal human tissues. Therefore, in the field of radiation therapy, how to reduce radiation pollution to an external environment, medical personnel or normal tissues of a patient while achieving effective treatment is an extremely important task.

EP 3 666 337 A1 discloses a neutron capture therapy system comprising an accelerator, a beam transmission device for guiding a charged particle beam, and a neutron beam generation device for producing a therapeutic neutron beam. The system is arranged in a facility having multiple chambers, such as an accelerator chamber, a beam transmission chamber, and an irradiation chamber, which are separated by partition walls or floors. In order to reduce radiation exposure outside the neutron beam generation region, the document proposes providing shielding assemblies at locations where system components pass through the partition walls or floors, and covering beam transmitting devices with shielding covers so as to suppress leakage of neutrons and other radiation into adjacent spaces.

EP 3 685 883 A1 discloses a neutron capture therapy system in which a neutron beam generation unit is configured to be movable between an operating position and a removal position to facilitate maintenance or replacement. The system includes a beam transmission portion comprising a vacuum tube connected to the neutron beam generation unit. A shielding device is provided to shield radiation from the neutron beam generation unit while it is being moved or removed, thereby reducing radiation exposure to operators during maintenance operations.

Therefore, it is required to propose a new technical solution to solve the described problems.

### SUMMARY

The invention is defined by independent claim 1. Further aspects of the invention are defined by the dependent claims.

In the neutron capture therapy system of the present disclosure, the beam transmission shielding assembly is provided, so as to reduce the secondary radiation and radiation damage caused by the recoil neutrons generated in the process of generating the neutron beam by the beam transmission portion and the neutron beam generation portion.

### BRIEF DESCRIPTION OF THE DRAWINGS

Fig. 1 is a schematic structural view of a neutron capture therapy system according to an embodiment of the present disclosure;
Fig. 2 is a schematic layout view of a neutron capture therapy system in an embodiment of the present disclosure; and
Fig. 3 is a schematic view of an embodiment of a transmission tube shielding member for a neutron capture therapy system in an embodiment of the present disclosure.

### DETAILED DESCRIPTION OF THE EMBODIMENTS

Embodiments of the present disclosure is further described in detail below with reference to the accompanying drawings, so thatthose skilled in the art may implement the present disclosure according to the text of the specification.

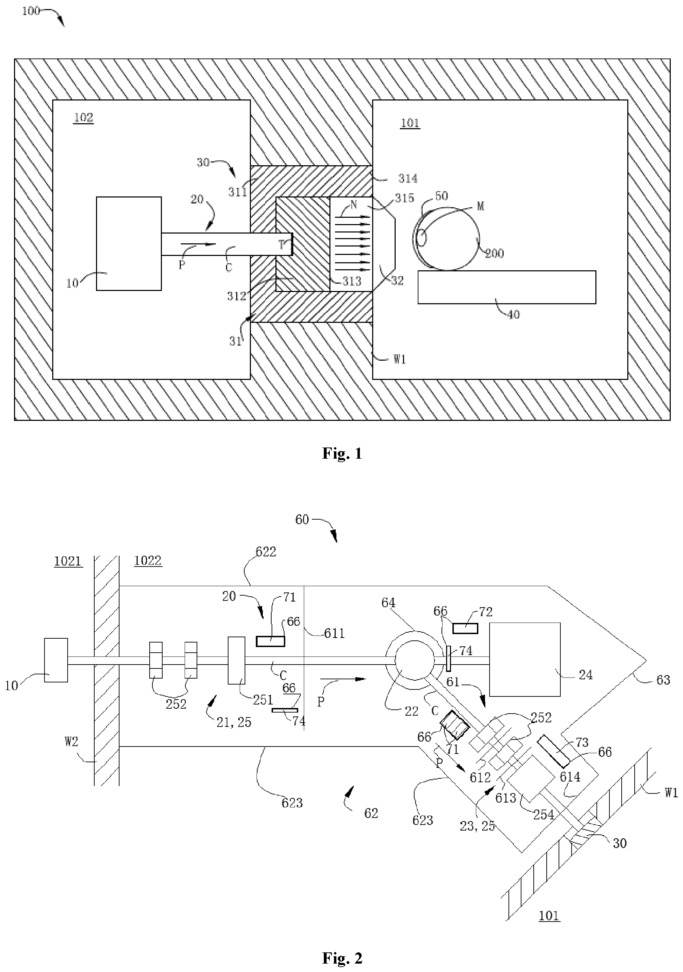

Referring to Fig. 1, a neutron capture therapy system in this embodiment is preferably a boron neutron capture therapy system 100. The boron neutron capture therapy system 100 is a device for performing cancer therapy by means of a boron neutron capture therapy. The boron neutron capture therapy carries out the cancer therapy by irradiating a neutron beam N to a patient 200 injected with boron (B-10). Upon taking or injecting the boron-containing (B-10) drug into the patient 200, the boron-containing drug selectively accumulates in tumor cells M. Based on a high capture cross section property for thermal neutrons of the boron-containing (B-10) drug, two heavy charged particles of ⁴He and ⁷Li are generated by a neutron capture and nuclear fission reaction of ¹⁰B(n, α)⁷Li. The average energy of the two charged particles of ⁴He and ⁷Li is about 2.33MeV, and the two charged particles have high Linear Energy Transfer (LET) and short distance properties, wherein the Linear Energy Transfer and distance of the alpha particle are 150keV/µm and 8µm, respectively, while that of the heavy particle of ⁷Li are 175keV/µm and 5µm, respectively, the total distance ofthe two heavy particles is approximately equivalentto a cell size, and therefore the radiation damage to organisms is limited to the cell level, which can achieve the purpose of locally killing tumor cells without causing too much damage to normal tissues.

The boron neutron capture therapy system 100 includes an accelerator 10, a beam transmission portion 20, a neutron beam generation portion 30, and a treatment station 40. The accelerator 10 is configured for accelerating the charged particles (such as protons, deuterons) to generate a charged particle beam P such as a proton beam; the beam transmission portion 20 transmits the charged particle beam P generated by the accelerator 10 to the neutron beam generation portion 30; the neutron beam generation portion 30 generates a neutron beam N for therapy and irradiates the patient 200 on the treatment station 40.

The neutron beam generation portion 30 includes a target T, a beam shaping portion 31, and a collimator 32. The charged particle beam P generated by the accelerator 10 irradiates the target T through the beam transmission portion 20 and interacts with the target T to generate neutrons. The generated neutrons form the neutron beam N for therapy through the beam shaping portion 31 and the collimator 32 in sequence, and irradiate the patient 200 on the treatment station 20. The target T is preferably a metal target. A suitable nuclear reaction is selected according to the required neutron yield and energy, energy and current of the available accelerated charged particles, and physical and chemical properties of the metal target, and the nuclear reactions commonly discussed are ⁷Li(p, n)⁷Be and ⁹Be(p, n)⁹B, which are both endothermic reactions. The energy threshold values of the two nuclear reactions are 1.881 MeV and 2.055 MeV, respectively, and since the ideal neutron source for the boron neutron capture therapy is epithermal neutrons of keV energy level, and theoretically, if the Li target is bombarded with protons whose energy is only slightly higherthan the energy threshold value, itis possible to generate neutrons of relatively low energy, which can be used in the clinic without too much slow speed treatment, however, the proton action cross sections of the two targets of lithium metal (Li) and beryllium metal (Be) with the threshold energy are not high, therefore protons of higher energy are usually chosen to initiate the nuclearreaction in orderto generate a sufficiently large flux of neutrons. An ideal target should have properties such as a high neutron yield, a distribution of energy ofthe generated neutron close to the energy region of the epithermal neutron (which will be described in detail below), no generation of too many long-distance radiation, low cost, safety, easy operation and high temperature resistance, but actually a nuclear reaction meeting all requirements cannot be found. As well known to a person skilled in the art, the target T may also be made of metal material other than Li and Be, such as Ta or W and an alloy thereof. The accelerator 10 may be a linear accelerator, a cyclotron, a synchrotron, a synchrocyclotron.

The beam shaping portion 31 may adjust the quality of the neutron beam N generated by the interaction of the charged particle beam P and the target T. The collimator 32 is configured for converging the neutron beam N, so that the neutron beam N has a higher targeting ability in the therapy process. The beam shaping portion 31 further includes a reflecting portion 311, a retarding portion 312, a thermal neutron absorbing portion 313, a radiation shielding portion 314, and a beam outlet 315. Neutrons generated by the interaction of the charged particle beam P and the target T have a wide energy spectrum, wherein in addition to the epithermal neutron meeting the therapy requirement, the proportion of other types of neutrons and photon needs to be minimized to avoid damage to the operator or the patient, therefore, the neutrons bombarded out from the target T need to pass through the retarding portion 312 to adjust the fast neutron energy (more than 40keV) therein to the epithermal neutron energy region (0.5 eV - 40keV) and minimize thermal neutrons (less than 0.5eV), the retarding portion 312 is made of a material having a larger action cross section with the fast neutron and a smaller action cross section with epithermal neutron, the retarding portion 312 is made of at least one of D₂O, AlF₃, Fluental^{™}, CaF₂, Li₂CO₃, MgF₂ and Al₂O₃. The reflecting portion 311 surrounds the retarding portion 312, reflects neutrons passing through the retarding portion 312 and diffusing around back into the neutron beam N so as to improve the utilization rate of neutrons, and is made of a material having strong neutron-reflecting capacity. In the embodiment, the reflecting portion 311 is made of at least one of Pb or Ni. The thermal neutron absorbing portion 313 is arranged behind the retarding portion 312, and is made of a material having a large action cross section with the thermal neutron. In the embodiment, the thermal neutron absorbing portion 313 is made of Li-6, the thermal neutron absorbing portion 313 is configured for absorbing the thermal neutrons passing through the retarding portion 312, so as to reduce the proportion of the thermal neutrons in the neutron beam N, thus avoiding excessive doses from being applied to superficial normal tissues during the therapy. It's understandable that the thermal neutron absorbing portion may be integrated with the retarding portion, the material of the retarding portion contains Li-6. The radiation shielding portion 314 is configured for shielding neutrons and photons leaked from parts other than the beam outlet 315. The material of the radiation shielding portion 314 includes at least one of photon shielding material and neutron shielding material. In the embodiment, the material of the radiation shielding portion 314 includes photon shielding material plumbum (Pb) and neutron shielding material polyethylene (PE). It's understandable that the beam shaping portion 31 may have other configurations, as long as the epithermal neutron beam required for therapy may be obtained. The collimator 32 is arranged behind the beam outlet 1315, the epithermal neutron beam coming out from the collimator 32 irradiates the patient 200, pass through the superficial normal tissues, and then is retarded thermal neutrons to reach the tumor cells M. It's understandable that the collimator 32 may be cancelled or replaced by other structures, in this case, the neutron beam comes out from the beam outlet 315 and directly irradiates the patient200. In the embodiment, a radiation shielding device 50 is further arranged between the patient 200 and the beam outlet 315, for shielding the radiation of the beam coming out from the beam outlet 315 to the normal tissues of the patient. It's understandable that the radiation shielding device 50 cannot be provided.

The target T is arranged between the beam transmission portion 20 and the beam shaping portion 31, and the beam transmission portion 20 has a transmission tube C for accelerating or transmitting the charged particle beam P. In the embodiment, the transmission tube C extends into the beam shaping portion 31 in the direction of the charged particle beam P, and sequentially passes through the reflecting portion 311 and the retarding portion 312, the target T is arranged in the retarding portion 312 and located at the end of the transmission tube C, so as to obtain a neutron beam with a better quality. Alternatively, the target may be arranged in other manners, for example, the target may be movable relative to the accelerator or the beam shaping portion, so as to facilitate replacement of the target or enable the charged particle beam to uniformly interact with the target.

The boron neutron capture therapy system 100 is housed entirely within a concrete-constructed building. The boron neutron capture therapy system 100 includes an irradiation chamber 101 and a charged particle beam generation chamber 102. The patient 200 on the treatment station 20 is performed therapy by means of irradiation of the neutron beam N in the irradiation chamber 101, the charged particle beam generation chamber 102 houses the accelerator 10 and at least part of the beam transmission portion 20. In conjunction with Fig. 2, the beam transmission portion 20 includes a first transmission portion 21 connected to the accelerator 10; a beam direction switcher 22 switching a travel direction of the charged particle beam P; and a second transmission portion 23 transmitting the charged particle beam P from the beam direction switcher 22 to the neutron beam generation portion 30. The generated neutron beam N irradiates the patient 200 in the irradiation chamber 101. The beam transmission portion 20 may further include a beam collector 24 configured for, such as, collecting the beam when it is not be required and confirming whether the charged particle beam P is outputted before performing the therapy. The beam direction switcher 22 enable the charged particle beam P to be deviated from a normal track thereof and leaded to the beam collector 24 and the beam direction switcher 22 can selectively transmit the charged particle beam P to the neutron beam generation portion 30 or the beam collector 24.

The charged particle beam generation chamber 102 may include an accelerator chamber 1021 and a beam transmission chamber 1022, the first transmission portion 21 extends from the accelerator chamber 1021 to the beam transmission chamber 1022, the second transmission portion 23 extends from the beam transmission chamber 1022 to the neutron beam generation portion 30, and the neutron beam generation portion 30 is at least partially arranged in a separation wall W1 between the beam transmission chamber 1022 and the irradiation chamber 101. The beam collector 24 is arranged in the beam transmission chamber 1022. It's understandable that the beam collector 24 may be arranged in other positions, such as in a concrete wall.

The beam direction switcher 22 may include a deflection electromagnet configured for deflecting the travel direction of the charged particle beam P and a switching electromagnet configured for controlling the transmission direction of the charged particle beam P. Each of the first transmission portion 21 and the second transmission portion 23 includes the transmission tube C, and may include a beam adjusting portion 25 arranged on the transmission tube C and configured for adjusting the charged particle beam P. The beam adjusting portion 25 includes an X-Y magnet 251 configured for adjusting the axes of the charged particle beam P, a quadrupole magnet 252 configured for suppressing the divergence of the charged particle beam P, a four-way septum magnet 253 (not shown) configured for shaping the charged particle beam P, and the like. The beam adjusting portion 25 ofthe second transmission portion 23 may further include a charged particle beam scanning magnet 254 configured for scanning the charged particle beam P as required to control the irradiation of the charged particle beam P with respect to the target T, for example, to control the irradiation position ofthe charged particle beam P with respectto the target T.

Since a large amount of neutrons may be generated during a neutron capture therapy, especially near the target T that generates neutrons, it is required to avoid the neutron leakage as much as possible. In one embodiment, the concrete forming at least part of the space (e. g., the beam transmission chamber 1022 and the irradiation chamber 101) is concrete added with neutron shielding material, such as boron-containing barite concrete, to form a neutron shielding space. In another embodiment, surfaces of concrete inside the chamber (such as the beam transmission chamber 1022, a ceiling plate, a floor plate and a wall of the irradiation chamber 101,) are disposed with neutron shielding plates (not shown) such as a boron-containing PE plate to form a neutron shielding space. The emission directions ofthe neutrons generated by the interaction ofthe charged particle beam P and the target T are uniformly distributed in space. During the process of "shaping" neutrons by the beam shaping portion 31, a large amount of recoil neutrons, which are needed to be focused on in a radiation shielding design, are generated. The recoil neutrons are mainly concentrated around the beam transmission portion 20 of the beam transmission chamber 1022, and the material of the beam transmission portion 20 (such as the transmission tube C and the magnet arranged on the transmission tube C) is generally made of steel-containing materials which, when irradiated by the neutrons, generates a radioisotope with a long half-life, causing secondary radiation, a beam transmission shielding assembly 60 for the beam transmission portion 20 therefore is provided, so as to reduce the secondary radiation and radiation damage caused by the recoil neutrons generated in the process of generating the neutron beam by the beam transmission portion 20 and the neutron beam generation portion 30.

The beam transmission shielding assembly 60 is arranged in the beam transmission chamber 102 and includes a magnet shielding member 61 and a transmission tube shielding member 62. The magnet shielding member 61 is arranged on the transmission tube C, and is located downstream of the beam adjusting portion 25 in the transmission direction of the charged particle beam P. In one embodiment, the magnet shielding member 61 is a shielding plate with a plate surface perpendicular to the transmission direction of the charged particle beam P. Each magnet may be provided with one shielding plate, alternatively, the downstream of the magnet at the most downstream of each transmission tube C in the transmission direction of the charged particle beam P may be provided with a shielding plate, and the material of the magnet shielding member 61 is boron- containing PE material. In the embodiment, the beam adjusting portion 25 of the first transmission portion 21 includes one X-Y magnet 251 and two quadrupole magnets 252, and the beam adjusting portion 25 of the second transmission portion 23 includes two quadrupole magnets 252 and one charged particle beam scanning magnet 254, the magnet shielding member 61 includes a shielding plate 611 arranged on the transmission tube C between the X-Y magnet 251 of the first transmission portion 21 and the beam direction switcher 22, shielding plates 612, 613 arranged between each two of magnets of the second transmission portion 23, and a shielding plate 614 arranged between the charged particle beam scanning magnet 254 of the second transmission portion 23 and the neutron beam generation portion 30. Since the second transmission portion 23 is closer to the neutron beam generation portion 30, more magnet shielding members 61 are provided.

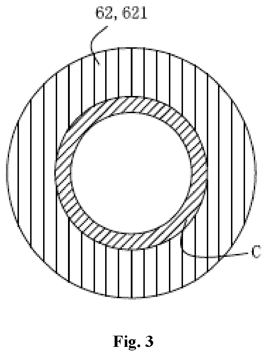

The transmission tube shielding member 62 is an annular shielding sleeve 621 (as shown in Fig. 3) surrounding the transmission tube C or includes shielding plates 622, 623 arranged at two sides of the transmission tube C in directions parallel to the transmission direction of the charged particle beam P. In order to reduce costs, the material of the transmission tube shield ing member 62 may be PE material without boron. In the case that the transmission tube shielding member 62 includes the shielding plates 622, 623 arranged at two sides of the transmission tube C in directions parallel to the transmission direction of the charged particle beam P, the transmission tube shielding member 62 provides additional shielding for both the magnets and other components on the beam transmission portion 20. In one embodiment, at least part of the magnetic shielding member 61 and the transmission tube shielding member 62 may be connected together and at least partially surround the beam adjusting portion 25 and the transmission tube C, so as to achieve a better shielding effect.

The beam transmission shielding assembly 60 further includes a shielding plate 63 arranged at an end of the beam collector 24, the shielding plate 63 at least partially surrounds the other end oppose to the end of the beam collector 24 connected to the beam direction switcher 22, and the cross section of the shielding plate 63 may be is a shape of [-shaped or polygonal-shaped. In the case that the transmission tube shielding member 62 includes the shielding plates 622, 623 arranged at two sides of the transmission tube C in directions parallel to the transmission direction of the charged particle beam P, in one embodiment, at least part of the magnet shielding member 61, the transmission tube shielding member 62 and the shielding plate 63 are connected together and form a closed space together with the separation wall W2 between the accelerator chamber 1021 and the beam transmission chamber 1022, so that the entire beam transmission portion 20 in the closed space is shielded. In the embodiment, the shielding plate 622 arranged at one side of the transmission tube C, the shielding plate 63 arranged at the end of the beam collector chamber 24, the shielding plate 614 arranged between the charged particle beam scanning magnet 254 of the second transmission portion 23 and the neutron beam generation portion 30, the shielding plate 623 arranged at the other side of the transmission tube C, the separation wall W2 between the accelerator chamber 1021 and the beam transmission chamber 1022 are sequentially connected to form a closed space. The shielding plate 611 arranged between the X-Y magnet 251 of the first transmission portion 21and the beam direction switcher 22 is connected to shielding plates 622 and 623 provided at two sides of the transmission tube C. Since the beam collector 24 contains a large mass of steel, in order to further reduce secondary radiation, a shielding cover (not shown) may be arranged at the top of the beam collector 24, alternatively, a shielding sleeve (not shown) may be arranged at the periphery of the beam collector 24 to surround the beam collector 24.

The beam direction switcher 22 may be surrounded by a shielding casing 64, so as to prevent the beam direction switcher 22 from interacting with the recoil neutrons to generate secondary radiation. The material of the shielding casing 64 may be boron-containing PE material.

The beam transmission shielding assembly 60 may further include a portable shielding plate 65 (not shown) which can be carried by an operator to move with the operator. The portable shielding plate 65 may further reduce radiation damage to the operator caused by secondary radiation generated by the beam transmission portion 20. It's understandable that the material of the portable shielding plate 65 is plumbum material or other photon shielding materials, and/or neutron shielding material.

The beam transmission portion 20 includes a transmission auxiliary device. The beam transmission shielding assembly 60 may further include an auxiliary shielding member 66 at least partially surrounding or covering the transmission auxiliary device. In the embodiment, the auxiliary shielding member 66 is a shielding box surrounding the transmission auxiliary device, and alternatively, a shielding casing or a shielding sleeve covering or surrounding the transmission auxiliary device. The auxiliary transmission device includes, but is not limited to, a first vacuum pump 71 and a second vacuum pump 72 configured for vacuuming, a control mechanism 73 (specifically, a water distribution tank) configured for controlling the on/off and flow rate of the cooling medium, and a control mechanism 74 (specifically, an argon tank) configured for controlling the on/off and flow rate of the argon gas. The material of the auxiliary shielding member 66 may be boron-containing PE material or other neutron shielding materials, and/or photon shielding material.

There may be one or more neutron beam generation portions 30, so as to generate one or more neutron beams N for therapy. Accordingly, the beam transmission portion 20 includes transmission portions transmitting the charged particle beams P to the neutron beam generation portions 30, while a same beam transmission shielding assembly 60 may be disposed for each of the transmission portions.

The boron neutron capture therapy system 100 may further include a preparation chamber, a control chamber, and other spaces configured for assisting the therapy.

It's understandable that, in this embodiment, the material of each of the concrete, the separation wall, the shielding plate, the shielding cover, the shielding sleeve, and the shielding casing may be replaced with other neutron shielding materials, and/or may be photon shielding materials; the shielding plate, the shielding cover, the shielding sleeve and the shielding casing may be mounted via an aluminum profile which, when irradiated by neutrons, generates a radioisotope with a short half-life. It's understandable that other materials can also be used. The shielding plate, the shielding cover, the shielding sleeve, and the shielding casing may be at least partially movable or detachable, which facilitates maintenance of the device.

## Claims

1. A neutron capture therapy system (100), comprising an accelerator (10), a beam transmission portion (20), and a neutron beam generation portion, wherein the accelerator (10) is configured for accelerating charged particles to generate a charged particle beam, the beam transmission portion (20) transmits the charged particle beam generated by the accelerator (10) to the neutron beam generation portion, and the neutron beam generation portion (30) generates a neutron beam for therapy, wherein
the neutron capture therapy system (100) comprises a beam transmission shielding assembly (60) for the beam transmission portion (20), wherein the beam transmission portion (20) comprises a transmission tube configured for accelerating or transmitting the charged particle beam and a beam adjusting portion (25) arranged on the transmission tube to adjust the charged particle beam, **characterized in that**
the beam transmission shielding assembly (60) comprises a second shielding member arranged on the transmission tube, and the second shielding member is located downstream of the beam adjusting portion (25) in a transmission direction of the charged particle beam; wherein the beam transmission shielding assembly (60) comprises third shielding members arranged at two sides of the transmission tube in the transmission direction of the charged particle beam, the second shielding member and the third shielding members are connected and at least partially surround the beam adjusting portion (25) and the transmission tube; wherein the third shielding members comprise shielding plates (622, 623) arranged at two sides
of the transmission tube in a direction parallel to the transmission direction of the charged particle beam.

2. The neutron capture therapy system according to claim 1, wherein the beam transmission shielding assembly (60) is at least partially movable or detachable.

3. The neutron capture therapy system according to claim 1, wherein the beam transmission portion (20) comprises a transmission tube configured for accelerating or transmitting the charged particle beam, and the beam transmission shielding assembly (60) comprises a first shielding member surrounding the transmission tube.

4. The neutron capture therapy system according to claim 1, wherein the second shielding member is a shielding plate (611, 612, 613) with a plate surface perpendicular to the transmission direction of the charged particle beam.

5. The neutron capture therapy system according to claim 1, wherein the beam adjusting portion (25) comprises an X-Y magnet (251) configured for adjusting axes of the charged particle beam, or a quadrupole magnet (252) configured for suppressing divergence of the charged particle beam, or a four-way septum magnet (253) configured for shaping the charged particle beam P, or a charged particle beam scanning magnet (254); and the second shielding member comprises at least one shielding plate located downstream of the charged particle beam scanning¹ magnet in the transmission direction of the charged particle beam.

6. The neutron capture therapy system according to claim 1, wherein the neutron capture therapy system comprises an irradiation chamber and a charged particle beam generation chamber, wherein a subject to be irradiated is performed therapy by means of irradiation of the neutron beam in the irradiation chamber, the charged particle beam generation chamber houses the accelerator (10) and at least part of the beam transmission portion (20), and the beam transmission shielding assembly (60) is arranged in the charged particle beam generation chamber.

7. The neutron capture therapy system according to claim 6, wherein the beam transmission portion (20) comprises a first transmission portion connected to the accelerator (10); a beam direction switcher switching a travel direction of the charged particle beam; and a second transmission portion transmitting the charged particle beam from the beam direction switcher to the neutron beam generation portion; and wherein the charged particle beam generation chamber comprises an accelerator chamber and a beam transmission chamber, the first transmission portion extends from the accelerator chamber to the beam transmission chamber, the second transmission portion extends from the beam transmission chamber (102) to the neutron beam generation portion, the neutron beam generation portion (30) is at least partially arranged in a separation wall between the beam transmission chamber (102) and the irradiation chamber, and the beam transmission shielding assembly (60) is arranged in the beam transmission chamber.

8. The neutron capture therapy system according to claim 7, wherein the beam transmission shielding assembly (60) comprises a shielding casing surrounding the beam direction switcher.

9. The neutron capture therapy system according to claim 7, wherein the beam transmission portion (20) comprises a beam collector, the beam direction switcher selectively transmits the charged particle beam to the neutron beam generation portion (30) or the beam collector; and wherein the beam transmission shielding assembly (60) comprises a first shielding plate arranged at an end of the beam collector, or a shielding cover arranged at a top of the beam collector, or a shielding sleeve surrounding the beam collector.

10. The neutron capture therapy system according to claim 9, wherein each of the first transmission portion and the second transmission portion comprises a transmission tube configured for accelerating or transmitting the charged particle beam and a beam adjusting portion (25) arranged on the transmission tube and configured for adjusting the charged particle beam; the beam transmission shielding assembly (60) comprises second shielding plates respectively arranged on transmission tubes of the first transmission portion and the second transmission portion and each having a plate surface perpendicular to a transmission direction of the charged particle beam, each of the second shielding plates is located downstream of corresponding beam adjusting portion; and the beam transmission shielding assembly (60) further comprises third shielding plates arranged at two sides of the first transmission portion and the second transmission portion in the transmission direction of the charged particle beam, and at least part of the second shielding plates, the first shielding plate and the third shielding plates are connected together and form a closed space together with the separation wall between the accelerator chamber and the beam transmission chamber.

11. The neutron capture therapy system according to claim 1, wherein the beam transmission portion (20) further comprises a transmission auxiliary device; and the beam transmission shielding assembly (60) comprises a forth shielding member at least partially surrounding or covering the transmission auxiliary device.

12. The neutron capture therapy system according to claim 1, wherein the beam transmission shielding assembly (60) further comprises a portable shielding plate.

## Patentansprüche

1. Ein Neutroneneinfangtherapiesystem (100), umfassend einen Beschleuniger (10), einen Strahlsendeteil (20) und einen Neutronenstrahlerzeugungsteil, wobei der Beschleuniger (10) zum Beschleunigen geladener Teilchen konfiguriert ist, um einen geladenen Teilchenstrahl zu erzeugen, der Strahlsendeteil (20) den durch den Beschleuniger (10) erzeugten geladenen Teilchenstrahl zu dem Neutronenstrahlerzeugungsteil sendet, und der Neutronenstrahlerzeugungsteil (30) einen Neutronenstrahl zur Therapie erzeugt, wobei das Neutroneneinfangtherapiesystem (100) eine Strahlsendeabschirmungsanordnung (60) für den Strahlsendeteil (20) umfasst, wobei der Strahlsendeteil (20) ein Senderohr, das zum Beschleunigen oder Senden des geladenen Teilchenstrahls konfiguriert ist, und einen Strahleinstellteil (25) umfasst, der an dem Senderohr angeordnet ist, um den geladenen Teilchenstrahl einzustellen, **dadurch gekennzeichnet, dass** die Strahlsendeabschirmungsanordnung (60) ein zweites Abschirmelement umfasst, das an dem Senderohr angeordnet ist, und das zweite Abschirmelement stromabwärts des Strahleinstellteils (25) in einer Senderichtung des geladenen Teilchenstrahls angeordnet ist; wobei die Strahlsendeabschirmungsanordnung (60) dritte Abschirmelemente umfasst, die an zwei Seiten des Senderohrs in der Senderichtung des geladenen Teilchenstrahls angeordnet sind, das zweite Abschirmelement und die dritten Abschirmelemente verbunden sind und den Strahleinstellteil (25) und das Senderohr mindestens teilweise umgeben; wobei die dritten Abschirmelemente Abschirmplatten (622, 623) umfassen, die an zwei Seiten des Senderohrs in einer Richtung parallel zu der Senderichtung des geladenen Teilchenstrahls angeordnet sind.

2. Das Neutroneneinfangtherapiesystem nach Anspruch 1, wobei die Strahlsendeabschirmungsanordnung (60) mindestens teilweise bewegbar oder abnehmbar ist.

3. Das Neutroneneinfangtherapiesystem nach Anspruch 1, wobei der Strahlsendeteil (20) ein Senderohr umfasst, das zum Beschleunigen oder Senden des geladenen Teilchenstrahls konfiguriert ist, und die Strahlsendeabschirmungsanordnung (60) ein erstes Abschirmelement umfasst, das das Senderohr umgibt.

4. Das Neutroneneinfangtherapiesystem nach Anspruch 1, wobei das zweite Abschirmelement eine Abschirmplatte (611, 612, 613) mit einer Plattenfläche senkrecht zu der Senderichtung des geladenen Teilchenstrahls ist.

5. Das Neutroneneinfangtherapiesystem nach Anspruch 1, wobei der Strahleinstellteil (25) einen X-Y-Magneten (251), der zum Einstellen von Achsen des geladenen Teilchenstrahls konfiguriert ist, oder einen Quadrupolmagneten (252), der zum Unterdrücken einer Divergenz des geladenen Teilchenstrahls konfiguriert ist, oder einen Vierwege-Septummagneten (253), der zum Formen des geladenen Teilchenstrahls P konfiguriert ist, oder einen Magneten (254) zum Scannen des geladenen Teilchenstrahls umfasst; und das zweite Abschirmelement mindestens eine Abschirmplatte umfasst, die in der Senderichtung des geladenen Teilchenstrahls stromabwärts des Magneten zum Scannen des geladenen Teilchenstrahls angeordnet ist.

6. Das Neutroneneinfangtherapiesystem nach Anspruch 1, wobei das Neutroneneinfangtherapiesystem eine Bestrahlungskammer und eine Kammer zur Erzeugung eines geladenen Teilchenstrahls umfasst, wobei an einem zu bestrahlenden Subjekt eine Therapie mittels Bestrahlung mit dem Neutronenstrahl in der Bestrahlungskammer durchgeführt wird, die Kammer zur Erzeugung eines geladenen Teilchenstrahls den Beschleuniger (10) und mindestens einen Teil des Strahlsendeteils (20) aufnimmt und die Strahlsendeabschirmungsanordnung (60) in der Kammer zur Erzeugung eines geladenen Teilchenstrahls angeordnet ist.

7. Das Neutroneneinfangtherapiesystem nach Anspruch 6, wobei der Strahlsendeteil (20) einen ersten Sendeteil, der mit dem Beschleuniger (10) verbunden ist; einen Strahlrichtungsschalter, der eine Laufrichtung des geladenen Teilchenstrahls schaltet; und einen zweiten Sendeteil umfasst, der den geladenen Teilchenstrahl von dem Strahlrichtungsschalter zu dem Neutronenstrahlerzeugungsteil sendet; und wobei die Kammer zur Erzeugung eines geladenen Teilchenstrahls eine Beschleunigerkammer und eine Strahlsendekammer umfasst, der erste Sendeteil sich von der Beschleunigerkammer zu der Strahlsendekammer erstreckt, der zweite Sendeteil sich von der Strahlsendekammer (102) zu dem Neutronenstrahlerzeugungsteil erstreckt, der Neutronenstrahlerzeugungsteil (30) mindestens teilweise in einer Trennwand zwischen der Strahlsendekammer (102) und der Bestrahlungskammer angeordnet ist, und die Strahlsendeabschirmungsanordnung (60) in der Strahlsendekammer angeordnet ist.

8. Das Neutroneneinfangtherapiesystem nach Anspruch 7, wobei die Strahlsendeabschirmungsanordnung (60) ein Abschirmgehäuse umfasst, das den Strahlrichtungsschalter umgibt.

9. Das Neutroneneinfangtherapiesystem nach Anspruch 7, wobei der Strahlsendeteil (20) einen Strahlsammler umfasst, der Strahlrichtungsschalter den geladenen Teilchenstrahl selektiv zu dem Neutronenstrahlerzeugungsteil (30) oder dem Strahlsammler sendet; und wobei die Strahlsendeabschirmungsanordnung (60) eine erste Abschirmplatte, die an einem Ende des Strahlsammlers angeordnet ist, oder eine Abschirmabdeckung, die an einer Oberseite des Strahlsammlers angeordnet ist, oder eine Abschirmhülse umfasst, die den Strahlsammler umgibt.

10. Das Neutroneneinfangtherapiesystem nach Anspruch 9, wobei jeder von dem ersten Sendeteil und dem zweiten Sendeteil ein Senderohr, das zum Beschleunigen oder Senden des geladenen Teilchenstrahls konfiguriert ist, und einen Strahleinstellteil (25) umfasst, der an dem Senderohr angeordnet und zum Einstellen des geladenen Teilchenstrahls konfiguriert ist; die Strahlsendeabschirmungsanordnung (60) zweite Abschirmplatten umfasst, die jeweils an Senderohren des ersten Sendeteils und des zweiten Sendeteils angeordnet sind und jeweils eine Plattenfläche senkrecht zu einer Senderichtung des geladenen Teilchenstrahls aufweisen, jede der zweiten Abschirmplatten stromabwärts eines entsprechenden Strahleinstellteils angeordnet ist; und die Strahlsendeabschirmungsanordnung (60) ferner dritte Abschirmplatten umfasst, die an zwei Seiten des ersten Sendeteils und des zweiten Sendeteils in der Senderichtung des geladenen Teilchenstrahls angeordnet sind, und mindestens ein Teil der zweiten Abschirmplatten, die erste Abschirmplatte und die dritten Abschirmplatten miteinander verbunden sind und zusammen mit der Trennwand zwischen der Beschleunigerkammer und der Strahlsendekammer einen geschlossenen Raum bilden.

11. Das Neutroneneinfangtherapiesystem nach Anspruch 1, wobei der Strahlsendeteil (20) ferner eine Sendehilfsvorrichtung umfasst; und die Strahlsendeabschirmungsanordnung (60) ein viertes Abschirmelement umfasst, das die Sendehilfsvorrichtung mindestens teilweise umgibt oder bedeckt.

12. Das Neutroneneinfangtherapiesystem nach Anspruch 1, wobei die Strahlsendeabschirmungsanordnung (60) ferner eine tragbare Abschirmplatte umfasst.

## Revendications

1. Un système de thérapie par capture de neutrons (100), comprenant un accélérateur (10), une partie de transmission de faisceau (20) et une partie de génération de faisceau de neutrons, dans lequel l'accélérateur (10) est configuré pour accélérer des particules chargées afin de générer un faisceau de particules chargées, la partie de transmission de faisceau (20) transmet le faisceau de particules chargées généré par l'accélérateur (10) à la partie de génération de faisceau de neutrons, et la partie de génération de faisceau de neutrons (30) génère un faisceau de neutrons pour une thérapie, dans lequel le système de thérapie par capture de neutrons (100) comprend un ensemble de blindage de transmission de faisceau (60) pour la partie de transmission de faisceau (20), dans lequel la partie de transmission de faisceau (20) comprend un tube de transmission configuré pour accélérer ou transmettre le faisceau de particules chargées et une partie d'ajustement de faisceau (25) agencée sur le tube de transmission pour ajuster le faisceau de particules chargées, **caractérisé en ce que** l'ensemble de blindage de transmission de faisceau (60) comprend un deuxième élément de blindage agencé sur le tube de transmission, et le deuxième élément de blindage est situé en aval de la partie d'ajustement de faisceau (25) dans une direction de transmission du faisceau de particules chargées ; dans lequel l'ensemble de blindage de transmission de faisceau (60) comprend des troisièmes éléments de blindage agencés sur deux côtés du tube de transmission dans la direction de transmission du faisceau de particules chargées, le deuxième élément de blindage et les troisièmes éléments de blindage sont reliés et entourent au moins partiellement la partie d'ajustement de faisceau (25) et le tube de transmission ; dans lequel les troisièmes éléments de blindage comprennent des plaques de blindage (622, 623) agencées sur deux côtés du tube de transmission dans une direction parallèle à la direction de transmission du faisceau de particules chargées.

2. Le système de thérapie par capture de neutrons selon la revendication 1, dans lequel l'ensemble de blindage de transmission de faisceau (60) est au moins partiellement mobile ou détachable.

3. Le système de thérapie par capture de neutrons selon la revendication 1, dans lequel la partie de transmission de faisceau (20) comprend un tube de transmission configuré pour accélérer ou transmettre le faisceau de particules chargées, et l'ensemble de blindage de transmission de faisceau (60) comprend un premier élément de blindage entourant le tube de transmission.

4. Le système de thérapie par capture de neutrons selon la revendication 1, dans lequel le deuxième élément de blindage est une plaque de blindage (611, 612, 613) ayant une surface de plaque perpendiculaire à la direction de transmission du faisceau de particules chargées.

5. Le système de thérapie par capture de neutrons selon la revendication 1, dans lequel la partie d'ajustement de faisceau (25) comprend un aimant X-Y (251) configuré pour ajuster des axes du faisceau de particules chargées, ou un aimant quadripolaire (252) configuré pour supprimer une divergence du faisceau de particules chargées, ou un aimant de septum à quatre voies (253) configuré pour façonner le faisceau de particules chargées P, ou un aimant de balayage de faisceau de particules chargées (254) ; et le deuxième élément de blindage comprend au moins une plaque de blindage située en aval de l'aimant de balayage du faisceau de particules chargées dans la direction de transmission du faisceau de particules chargées.

6. Le système de thérapie par capture de neutrons selon la revendication 1, dans lequel le système de thérapie par capture de neutrons comprend une chambre d'irradiation et une chambre de génération de faisceau de particules chargées, dans lequel un sujet à irradier est soumis à une thérapie au moyen d'une irradiation par le faisceau de neutrons dans la chambre d'irradiation, la chambre de génération de faisceau de particules chargées abrite l'accélérateur (10) et au moins une partie de la partie de transmission de faisceau (20), et l'ensemble de blindage de transmission de faisceau (60) est agencé dans la chambre de génération de faisceau de particules chargées.

7. Le système de thérapie par capture de neutrons selon la revendication 6, dans lequel la partie de transmission de faisceau (20) comprend une première partie de transmission reliée à l'accélérateur (10) ; un commutateur de direction de faisceau commutant une direction de déplacement du faisceau de particules chargées ; et une deuxième partie de transmission transmettant le faisceau de particules chargées depuis le commutateur de direction de faisceau jusqu'à la partie de génération de faisceau de neutrons ; et dans lequel la chambre de génération de faisceau de particules chargées comprend une chambre d'accélérateur et une chambre de transmission de faisceau, la première partie de transmission s'étend depuis la chambre d'accélérateur jusqu'à la chambre de transmission de faisceau, la deuxième partie de transmission s'étend depuis la chambre de transmission de faisceau (102) jusqu'à la partie de génération de faisceau de neutrons, la partie de génération de faisceau de neutrons (30) est agencée au moins partiellement dans une paroi de séparation entre la chambre de transmission de faisceau (102) et la chambre d'irradiation, et l'ensemble de blindage de transmission de faisceau (60) est agencé dans la chambre de transmission de faisceau.

8. Le système de thérapie par capture de neutrons selon la revendication 7, dans lequel l'ensemble de blindage de transmission de faisceau (60) comprend un boîtier de blindage entourant le commutateur de direction de faisceau.

9. Le système de thérapie par capture de neutrons selon la revendication 7, dans lequel la partie de transmission de faisceau (20) comprend un collecteur de faisceau, le commutateur de direction de faisceau transmet sélectivement le faisceau de particules chargées à la partie de génération de faisceau de neutrons (30) ou au collecteur de faisceau ; et dans lequel l'ensemble de blindage de transmission de faisceau (60) comprend une première plaque de blindage agencée à une extrémité du collecteur de faisceau, ou un capot de blindage agencé sur un sommet du collecteur de faisceau, ou un manchon de blindage entourant le collecteur de faisceau.

10. Le système de thérapie par capture de neutrons selon la revendication 9, dans lequel chacune de la première partie de transmission et de la deuxième partie de transmission comprend un tube de transmission configuré pour accélérer ou transmettre le faisceau de particules chargées et une partie d'ajustement de faisceau (25) agencée sur le tube de transmission et configurée pour ajuster le faisceau de particules chargées ; l'ensemble de blindage de transmission de faisceau (60) comprend des deuxièmes plaques de blindage respectivement agencées sur des tubes de transmission de la première partie de transmission et de la deuxième partie de transmission et ayant chacune une surface de plaque perpendiculaire à une direction de transmission du faisceau de particules chargées, chacune des deuxièmes plaques de blindage est située en aval de la partie d'ajustement de faisceau correspondante ; et l'ensemble de blindage de transmission de faisceau (60) comprend en outre des troisièmes plaques de blindage agencées sur deux côtés de la première partie de transmission et de la deuxième partie de transmission dans la direction de transmission du faisceau de particules chargées, et au moins une partie des deuxièmes plaques de blindage, la première plaque de blindage et les troisièmes plaques de blindage sont reliées ensemble et forment un espace fermé conjointement avec la paroi de séparation entre la chambre d'accélérateur et la chambre de transmission de faisceau.

11. Le système de thérapie par capture de neutrons selon la revendication 1, dans lequel la partie de transmission de faisceau (20) comprend en outre un dispositif auxiliaire de transmission ; et l'ensemble de blindage de transmission de faisceau (60) comprend un quatrième élément de blindage entourant ou couvrant au moins partiellement le dispositif auxiliaire de transmission.

12. Le système de thérapie par capture de neutrons selon la revendication 1, dans lequel l'ensemble de blindage de transmission de faisceau (60) comprend en outre une plaque de blindage portable.
